(19) Europäisches Patentamt
European Patent Office
Office européen des brevets

(11) EP 0 486 153 B1

(12) EUROPEAN PATENT SPECIFICATION

(45) Date of publication and mention of the grant of the patent:
03.01.1996 Bulletin 1996/01

(21) Application number: 91309312.6

(22) Date of filing: 10.10.1991

(51) Int Cl.[6]: C12P 17/06, C12P 7/42, C12P 7/62, C12N 9/14, C12N 11/00 // (C12P17/06;, C12R1:645)

(54) **Enzymatic hydrolysis of lovastatin acid using an enzyme derived from Clonostachys compactiuscula**

Enzymatische Hydrolyse der Lovalovastatinsäure durch ein von Clonostachys compactiuscula stammendes Enzym

Hydrolyse enzymatique de l'acide lovastatinique par une enzyme dérivée de Clonostachys compactiuscula

(84) Designated Contracting States:
CH DE FR GB IT LI NL

(30) Priority: 15.10.1990 US 597643

(43) Date of publication of application:
20.05.1992 Bulletin 1992/21

(73) Proprietor: MERCK & CO. INC.
Rahway, New Jersey 07065-0900 (US)

(72) Inventors:
- Conder, Michael J.
  Harrisonburg, VA 22801 (US)
- Cover, William H.
  McGaheysville, VA 22840 (US)
- Dabora, Rebecca L.
  Andover, MA 01810 (US)
- Stieber, Robert W.
  Harrisonburg, VA 22801 (US)
- Tehlewitz, Bogdan
  McGaheysville, VA 22840 (US)

(74) Representative: Cole, William Gwyn et al
Harlow, Essex CM20 2QR (GB)

(56) References cited:
- PATENT ABSTRACTS OF JAPAN vol. 10, no. 23 (C-325)(2080) 29 January 1986
- PATENT ABSTRACTS OF JAPAN vol. 9, no. 287 (C-314)(2010) 14 November 1985
- PATENT ABSTRACTS OF JAPAN vol. 5, no. 12 (C-40)(684) 24 January 1981

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

**Description**

BRIEF SUMMARY OF THE INVENTION

The present invention relates to biosynthetic production of 6(R)-[2-(8(S)-hydroxy-2(S), 6(R)-dimethyl-1,2,6,7,8,8a (R)-hexahydronaphthyl)-ethyl]-4(R)-hydroxy-3,4,5,6-tetrahydro-2H-pyran-2-one triol acid (2) by microbiological hydrolysis of lovastatin acid, a fermentation product, using Clonostachys compactiuscula, or a hydrolase derived therefrom.

The triol acid and its lactone form are old compounds, i. e., ones known in the art, and they are inhibitors of 3-hydroxy-3-methylglutaryl-coenzyme A (HMG-CoA) reductase, an enzyme involved in cholesterol biosynthesis. As inhibitors of that enzyme, they are useful as antihypercholesterolemic agents. They find further usefulness as intermediates for the preparation of other antihypercholesterolemic agents, especially those having various side chains at the 8-position. For example, simvastatin, which has a 2,2-dimethylbutyryloxy side chain at the 8-position, may be prepared using the lactone form of the triol acid as a starting material, in accordance with known procedures.

The present invention also relates to a substantially pure form of the hydrolase enzyme produced by Clonostachys compactiuscula ATCC 38009, and mutants thereof, which is capable of transforming lovastatin acid or a salt thereof to triol acid in accordance with the process of the present invention.

The present invention further relates to a process in which the triol acid produced by treating lovastatin acid or a salt thereof with Clonostachys compactiuscula ATCC 38009, or mutants thereof, or a hydrolase derived therefrom, is thereafter converted to its lactone form in accordance with conventional procedures well known in the art. ATCC 38009 was deposited with the American Type Culture Collection, 12301 Parklawn Drive, Rockville, Maryland, USA, on Ist December, 1978.

BACKGROUND OF THE INVENTION

The present invention is in the field of inhibitors of HMG-CoA reductase which are useful as antihypercholesterolemic agents. It is now well established that hypercholesterolemia is a significant risk factor in the development of cardiovascular disease, particularly atherosclerosis. Compounds which are able to inhibit the HMG-CoA reductase enzyme interfere with and limit the biosynthesis of cholesterol, and in that way function as antihypercholesterolemic agents. Such compounds, especially the natural fermentation products compactin and mevinolin, are now well known. There is a continuous search, nevertheless, for additional semisynthetic analogs which will give improved antihypercholesterolemic performance. The triol acid produced by enzymatic hydrolysis of lovastatin acid using an enzyme derived from Clonostachys compactiuscula in accordance with the biosynthetic process of the present invention provides quantities of a starting material for the preparation and production of such semisynthetic analogs.

As already described above, the triol acid and its lactone form are old compounds. The triol acid in its lactone form, for example, is described in Endo, published Japanese Pat. Appln. 86-13798 (1986), where its production by fermentation of Monascus ruber and a demonstration of its ability to reduce blood cholesterol levels is also set out. The triol acid in its lactone form, as well as the triol acid itself, are also described in Willard U.S. Pat. No. 4,293,496 (1981). However, there these compounds are prepared by chemical hydrolysis to remove the 8-(α-methylbutyryloxy) ester side chain of a starting material which is a fermentation product of a particular strain of Aspergillus terreus. There is no suggestion that such hydrolysis might be carried out microbiologically.

Komagata et al., J. Antibiotics, 39, 1574-77 (1986), describes enzymatic hydrolysis conversion of ML-236B to ML-236A in which the same side chain is removed as in the present invention. Of 1600 fungal strains investigated, 59 were found to be effective in catalyzing the hydrolytic reaction, and Emericella unguis showed the most potent activity. However, C. compactiuscula is not disclosed.

Endo, published Japanese Pat. Appln. 85-176595 (1985) describes the same conversion as Komagata et al. above, but additionally includes conversion of "monacolin K" to "monacolin J", which is the same as the conversion of lovastatin to triol acid in the present invention. Especially useful are said to be the molds Mortierella isabellina, Emericella unguis, Diheterospora chlamydosporia, Humicola fuscoatra, Dichotomomyces cejpii, Neocosmospora africana, Xylogone sphaerospora, Torulomyces ragena, and Thielavia fimeti. However, the highest conversion rate is 78% for a starting material concentration of 0.5 mg/ml, compared to 80-90% with the present invention. And, there is an indication in the related Komagata et al. paper that at higher concentrations, such as the 2.5 mg/ml employed in the present invention, there is a significant drop off in efficiency of the enzyme. Thus, there is no suggestion in the prior art of the improved microbiological hydrolysis which can be achieved using Clonostachys compactiuscula.

DETAILED DESCRIPTION OF THE INVENTION

The present invention is concerned with biosynthetic production of 6(R)-[2-(8(S)-hydroxy-2(S), 6(R) -dimethy1-1,2,6,7,8,8a(R)-hexahydronaphthyl)-ethyl]-4(R)-hydroxy-3,4,5,6-tetrahydro-2H-pyran-2-one triol acid by

treating lovastatin acid (1) or a salt thereof with Clonostachys compactiuscula or mutants thereof, or a hydrolase derived therefrom. The triol acid may be subsequently converted by known chemistry to its lactone form.

The starting material for the method of the present invention is lovastatin acid (1), the open-ring form of lovastatin, or a salt thereof. The acid form is the material produced by fermentation of Aspergillus terreus in accordance with culturing methods known in the art. Lovastatin itself is too insoluble in aqueous systems to be a useful starting material in the method of the present invention; and those solvents in which it is soluble are generally incompatible with the method of the present invention.

The lovastatin acid starting material will typically be employed in the salt form, and the term "lovastatin acid" unless otherwise specified, is meant to include any suitable salt form thereof as well. Any salt which permits good solubility and which will not interfere with the other conditions encountered in carrying out the method of the present invention is permissible. For example, the alkali metal salts, such as Na and K, may be employed. The ammonium salt form may be employed and is preferred.

For convenience, the structural formulas for lovastatin acid, the triol acid, and its lactone form, are set out below as Formulas 1, 2, and 3, respectively:

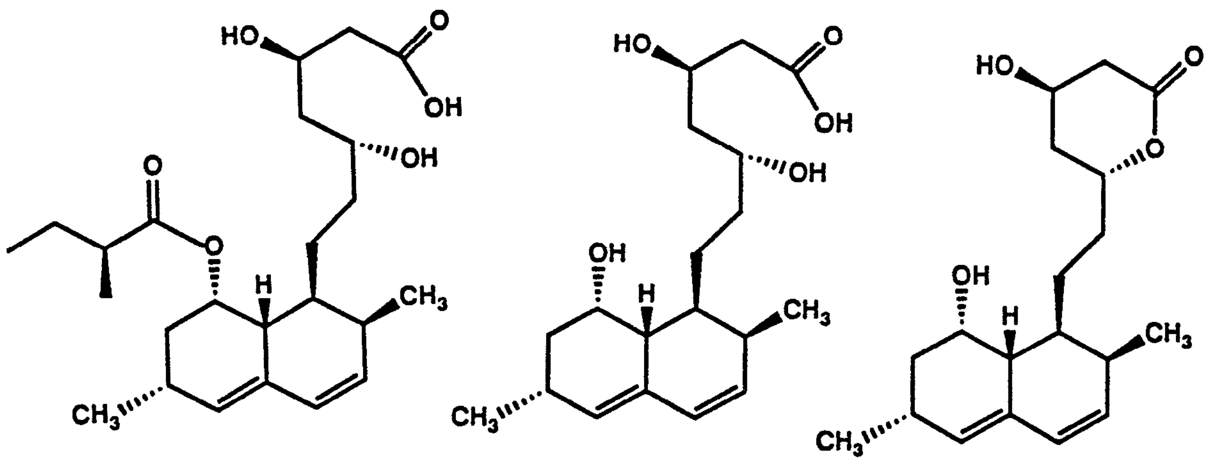

1 Lovastatin Acid    2 Triol Acid    3 Diol Lactone

The basic mechanism of biosynthetic production of triol acid in accordance with the present invention is thought to be enzymatic hydrolysis of lovastatin acid whereby an enzyme produced by Clonostachys compactiuscula ATCC 38009, or mutants thereof, catalyzes removal of the 8-(α-methylbutyryloxy) ester side chain of lovastatin acid to give the triol acid. As already explained, for reasons of solubility in aqueous systems, it has been found most desirable to use the lovastatin starting material in its open ring or acid form, and for this purpose the ammonium salt form of lovastatin acid is preferred.

The enzyme produced by Clonostachys compactiuscula may be brought into contact with the lovastatin acid starting material in any number of ways, all of which will be apparent to the person of ordinary skill in this art. This is the definition of the term "treating" in its broadest context. For example, whole cells may be used, and in accordance with this procedure, a fermentation culture of Clonostachys compactiuscula is produced to which the lovastatin acid starting material is simply added and the triol acid final product recovered.

A variation of this whole cell procedure is one in which a fermentation culture of Clonostachys compactiuscula as described above is produced, but a smaller concentration of lovastatin acid is added for the purpose of inducing hydrolytic activity. The cell mass is then harvested and recovered as pellets which can be used immediately or frozen for later use. These may be added to the lovastatin acid starting material where the latter is present in the fermentation culture in which is has been produced, e.g., by fermentation of Aspergillus terreus. Alternatively, the lovastatin acid may be separated from its culture medium and then brought into contact with the frozen pellets of Clonostachys compactiuscula described above.

It is not necessary that the whole cells of Clonostachys compactiuscula be alive as described above. It is also possible to employ dead cells, e.g., those which have been acetone-dried.

As an alternative to whole cells, it is possible to use crude homogenates derived from these whole cell cultures. It is also possible to isolate the hydrolytic enzyme itself from the crude homogenates and employ it.

The process of bringing the Clonostachys compactiuscula enzyme into contact with the lovastatin acid starting material may be carried out batch-wise, or it may be carried out in a continuous manner. The contacting of these reactants themselves may be modified in various ways in keeping with advances in process technology. Thus, an immobilized

enzyme column may be employed for the Clonostachys compactiuscula enzyme with the lovastatin acid starting material being passed through the column. Another example of such process technology is that relating to membrane reactors. Another alternative process for contacting of the reactants would be to culture the Clonostachys compactiuscula ATCC 38009, or mutants, in the same fermentation broth used to produce the lovastatin. It would also be possible to modify that fermentation broth, if necessary, in order to support growth of Clonostachys compactiuscula once the lovastatin acid is produced, by adding culture media elements and then introducing the Clonostachys compactiuscula ATCC 38009, or mutants, and culturing it to produce the triol acid. This approach, however, is not likely to produce optimum yields. The preferred method of contacting the reactants is by way of the immobilized enzyme column described above.

Working examples set out further below describe the method currently employed to demonstrate the enzymatic hydrolysis of lovastatin acid. However, the methods in those working examples would not necessarily be suggestive of methods which would be utilized for commercial production.

The present invention is also directed to the use of mutants of the particular strain of Clonostachys compactiuscula, ATCC 38009, which are capable of converting lovastatin acid to triol acid. There are techniques well known in the fermentation art for improving the yields of desired products produced by various strains of microorganisms. For example, a given producing strain may be irradiated or exposed to other stimuli known to greatly increase the ongoing mutation of the genetic material of the microorganism. By using a sensitive screen, it is then possible to select from the many mutations thus produced only those which result in an enhanced production of the desired product. In this way, it is usually possible to continually improve the output of a producing strain through its various selected descendants. With regard to the present invention, similar improvements in output of lovastatin acid hydrolase by selected mutants of Clonostachys compactiuscula ATCC 38009, may be achieved. A satisfactory screen for this purpose is the use of high performance liquid chromatography (HPLC) which can detect the enzymatic cleavage products at very low concentrations, thus clearly establishing that triol acid has been produced by any particular mutant in question.

## Culture Medium

The fermentation of Clonostachys compactiuscula is carried out in aqueous media such as those employed for the production of other fermentation products. Such media contain sources of carbon, nitrogen and inorganic salts assimilable by the microorganism.

In general, carbohydrates such as sugars, for example, lactose, glucose, fructose, maltose, sucrose, xylose, mannitol and the like and starches such as grains, for example, oats, ryes, cornstarch, corn meal and the like can be used either alone or in combination as sources of assimilable carbon in the nutrient medium. The exact quantity of the carbohydrate source or sources utilized in the medium depends in part upon the other ingredients of the medium but, in general, the amount of carbohydrate usually varies between about 1% and 6% by weight of the medium. These carbon sources can be used individually, or several such carbon sources may be combined in the medium. In general many proteinaceous materials may be used as nitrogen sources in the fermentation process. Suitable nitrogen sources include for example, yeast hydrolysates, primary yeast, soybean meal, cottonseed flour, hydrolysates of casein, corn steep liquor, distiller's solubles or tomato paste and the like. The sources of nitrogen either alone or in combination, are used in amounts ranging from about 0.2% to 6% by weight of the aqueous medium.

Among the nutrient inorganic salts which can be incorporated in the culture media are the customary salts capable of yielding sodium, potassium, ammonium, calcium, phosphate, sulfate, chloride, carbonate, and like ions. Also included are trace metals such as cobalt, manganese, iron and magnesium.

It should be noted that the media described in the Examples are merely illustrative of the wide variety of media which may be employed, and yet are not intended to be limitative. Specifically, the carbon sources used in the culture media to produce triol acid include dextrose, dextrin, oat flour, oatmeal, molasses, citrate, soybean oil, glycerol, malt extract, cod liver oil, starch, ethanol, figs, sodium ascorbate and lard oil. Included as nitrogen sources were peptonized milk, autolyzed yeast, yeast RNA, tomato paste, casein, primary yeast, peanut meal, distillers solubles, corn steep liquor, soybean meal, corn meal, NZ amine, bean extract, aspargine, cottonseed meal an ammonium sulfate. The major ionic components are $CaCO_3$, $KH_2PO_4$, $MgSO_4 \cdot 7H_2O$ and NaCl and small amounts of $CoCl_2 \cdot 6H_2O$ and traces of Fe, Mn, Mo, B, Co and Cu were also present.

## Lactonization

Treatment of lovastatin acid with Clonostachys compactiuscula ATCC 38009, or mutants thereof, or a hydrolase derived therefrom, in accordance with the process of the present invention provides the triol acid as the predominant product. However, it is also desirable to obtain the lactone form of this compound, since it is also useful as an antihypercholesterolemic agent or as an intermediate for preparing such agents. Lactonization of triol acid is carried out using standard procedures, i.e., either heat or acid catalyzed lactonization. Procedures for acid-catalyzed lactonization of

lovastatin acid-related compounds is described in U.S. Patent 4,916,239. For triol acid, lactonization has been carried out by stirring in isopropyl acetate containing 7mM methane sulfonic acid for 2 hrs at room temperature.

EXAMPLE 1

Biotransformation of lovastatin acid to triol acid by whole cells of Clonostachys compactiuscula

Clonostachys compactiuscula ATCC 38009 was grown in a 2L airlift fermentor with 1.8 L working volume in medium EN (glucose 1%; peptone 0.2%; beef extract 0.1%; yeast extract 0.1%; and corn steep liquor 0.3%), at 29°C, at an aeration rate of 1.25 vvm, for 48-72 hrs. Lovastatin ammonium salt was added (0.5 g/L final concentration) to induce hydrolytic activity. The fermentation was harvested 24-72 hrs. after addition of the lovastatin ammonium salt by straining through a sieve and washing the pellets with buffer (20 mM Tris, pH 8.5). The cell pellets were frozen until ready to use.

For the biotransformation, Clonostachys compactiuscula pellets (17 g wet weight) from an airlift fermentation were contacted with 20 ml of crude lovastatin acid (20 g/L) in carbonate buffer harvested from an Aspergillus terreus fermentation. The biotransformation was carried out in a 250 ml Erlenmeyer flask at 27° C and 160 rpm.
After 17 hrs. approximately 60% of the lovastatin acid was converted to triol acid.

In an additional experiment, Clonostachys compactiuscula pellets from an airlift fermentation (5 g wet weight) were contacted with 10 ml crude lovastatin acid (3.5 g/L) extracted from an A terreus fermentation by methanol. The final concentration of methanol in the biotransformation mixture was 25%. The bioreaction was carried out in a 250 ml Erlenmeyer flask at 27° C and 160 rpm. After 2 hrs. the biotransformation employing Clonostachys compactiuscula converted nearly 100% of the lovastatin acid to triol acid, as measured by thin layer chromatography.

EXAMPLE 2

Biotransformation of lovastatin acid to triol acid by crude homogenate of Clonostachys compactiuscula

Clonostachys compactiuscula ATCC 38009 was grown in 250 ml shake flasks containing 12 ml of medium EN at 29° C for 3 days. Lovastatin ammonium salt was added to give a concentration of 0.5 g/L and fermentation was continued for 2 additional days. To prepare the crude homogenate, the culture was harvested by centrifugation at 3000 rpm for 10 minutes, after which it was washed with 50 mM of N-tris(hydroxymethyl)methyl-2-aminoethanesulfonic acid (TES) buffer, pH 7.7. The culture medium was again centrifuged and the cell mass was chilled on ice and then subjected to grinding in a mortar and pestel containing glass fragments and powdered dry ice. The contents of 1 shake flask was resuspended in 2.0 ml of 50 mM TES buffer and centrifuged at 2000 rpm for 10 minutes to remove cell debris and glass fragments. The supernatant was used as the source of crude homogenate with protein concentration of approximately 0.5 mg/ml.

In order to carry out the biotransformation, one volume of crude homogenate was combined with an equal volume of lovastatin acid ammonium salt (5 g/L), and the mixture was incubated at 29° C. Using this method, 80-90% conversion of lovastatin acid to triol acid was observed within 2 hrs.

EXAMPLE 3

Biotransformation of lovastatin acid to triol acid by purified hydrolase from Clonostachys compactiuscula

A hydrolytic enzyme which carries out the biotransformation of lovastatin acid to triol acid was purified by Fast Protein Liquid Chromatography (FPLC®) employing a Mono Q® anion exchange column to near homogeneity from homogenates of Clonostachys compactiuscula employing the procedures described below.

A supernatant from the 2,000 rpm centrifugation as in Example 2 above, but where 20 mM of tromethamine (TRIS) buffer is substituted for 50 mM TES buffer, was centrifuged at 15,000 rpm and the resulting supernatant filtered through a 0.45 μm filter. Batches (10 ml) of filtrate containing 0.3-0.5 mg/ml protein were then applied to a Pharmacia Mono Q® anion exchange column connected to a Pharmacia Fast Protein Liquid Chromatography (FPLC®) system.

After allowing binding of the anionic proteins to the column matrix, the hydrolase was specifically eluted by the application of a linear gradient of sodium chloride (0-500mM). Eluted protein was collected in 1 ml fractions and assayed either using lovastatin ammonium salt (in which case percent hydrolysis was estimated by TLC and densitometry or HPLC), or a colorimetric substrate (ortho-nitrophenyl butyrate o-NPB) towards which the enzyme had been shown to have hydrolytic activity. When the latter substrate was used, the hydrolytic reaction was monitored spectrophotometrically at 410 nm essentially as described by Lawrence, R.C. et al. in J. Gen. Microbiol. (1967) 48, 401-418. Both assay methods revealed that the hydrolase was eluted when the NaCl concentration approached 300 mM.

Sodium dodecyl sulfate-polyacrylamide gel electrophoresis revealed the peak lovastatin acid hydrolase-containing fractions to contain a prominant band of molecular weight approximately 45,000 Da.

Using the purified enzyme preparation, the biotransformation was carried out in accordance with the procedures described above in Examples 1 and 2, and an estimate was made of the hydrolase's Km and specific activity with lovastatin ammonium salt as substrate. The value for Km obtained was 4.14mM and under saturating substrate conditions the enzyme was found to have a specific activity of 0.11mmol lovastatin ammonium salt/mg protein per hour.

**Claims**

1. A process for preparing a compound of Formula 2,

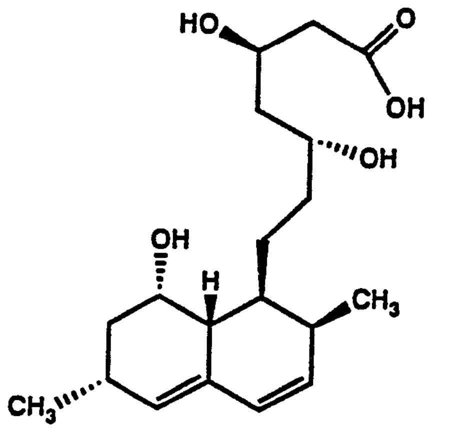

2 Triol Acid

or a salt thereof in recoverable amounts thereof comprising treating a compound of Formula 1

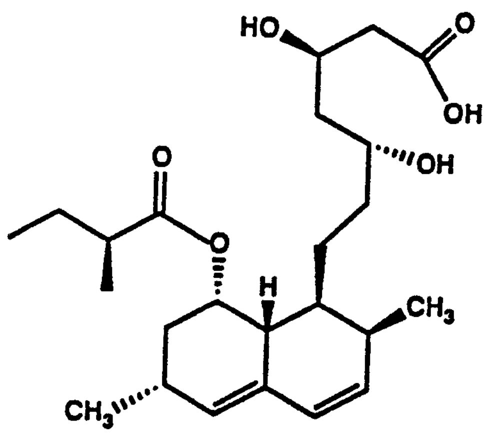

1 Lovastatin Acid

or a salt thereof with Clonostachys compactiuscula ATCC 38009, or mutants thereof, or a hydrolase derived therefrom, and recovering the product.

2. The process according to Claim 1 wherein the salt is the ammonium salt.

3. The process according to Claim 1 wherein the hydrolase is in purified form and immobilized on a column, and the compound of Formula 1 is brought into contact therewith by passage through said column.

4. A substantially pure form of the hydrolase enzyme produced by Clonostachys compactiuscula ATCC 38009, and mutants thereof, which is capable of carrying out a process according to Claim 1.

5. The process of Claim 1 further comprising lactonization of the triol acid of Formula 2 to provide a diol lactone of structural Formula 3:

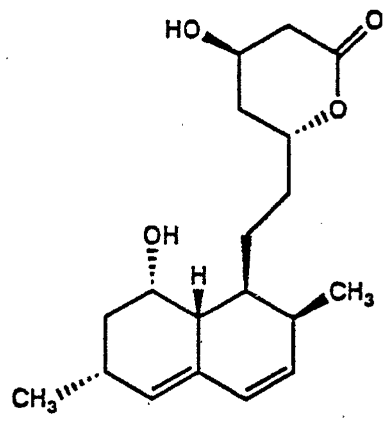

3 Diol lactone

**Patentansprüche**

1. Ein Verfahren zur Herstellung einer Verbindung der Formel 2

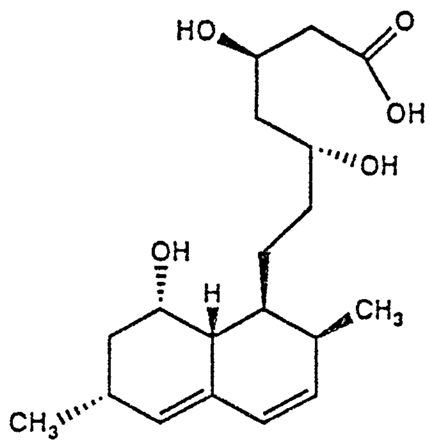

2 Triolsäure

oder eines Salzes davon in gewinnbaren Mengen davon, das die Behandlung einer Verbindung der Formel 1

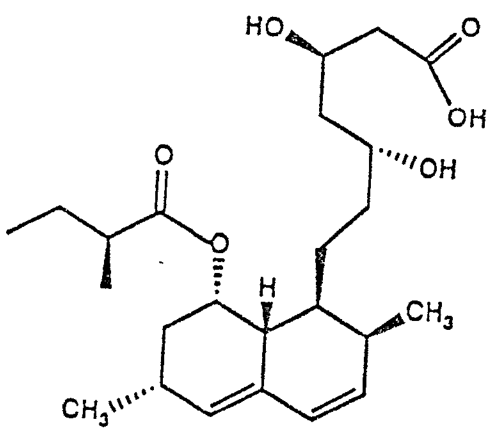

1 Lovastatinsäure

oder eines Salzes davon mit Clonostachys compactiuscula ATCC 38009 oder Mutanten davon oder einer davon abstammenden Hydrolase und die Gewinnung des Produkts umfaßt.

2. Das Verfahren nach Anspruch 1, worin das Salz das Ammoniumsalz ist.

3. Das Verfahren nach Anspruch 1, worin die Hydrolase in gereinigter Form und auf einer Säule immobilisiert vorliegt, und die Verbindung der Formel 1 mit ihr durch Durchfluß durch die Säule in Kontakt gebracht wird.

4. Eine im wesentlichen reine Form des Hydrolaseenzyms, hergestellt durch Clonostachys compactiuscula ATCC 38009 und Mutanten davon und in der Lage, ein Verfahren nach Anspruch 1 auszuführen.

5. Das Verfahren nach Anspruch 1, das ferner die Lactonisierung der Triolsäure der Formel 2 umfaßt, um ein Diollacton der Strukturformel 3:

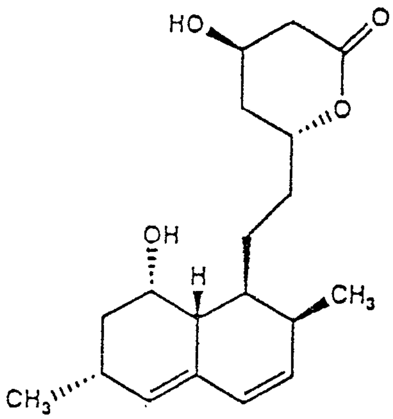

3 Diollacton

zu ergeben.

**Revendications**

1. Procédé pour la préparation d'un composé de formule 2

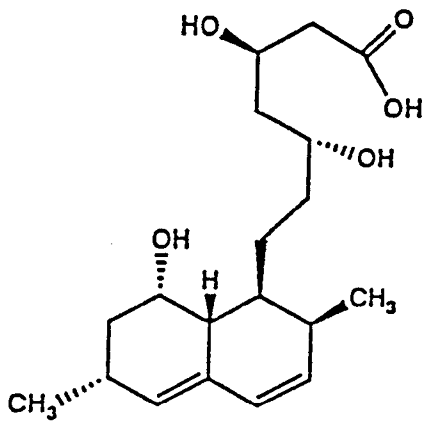

2 acide triol

ou d'un sel de celui-ci en des quantités récupérables, comprenant le traitement d'un composé de formule 1

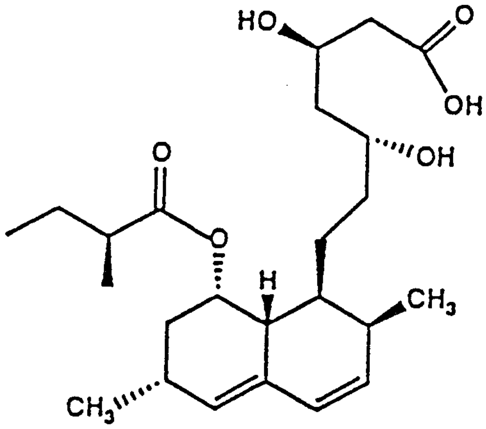

1 acide lovastatinique

ou d'un sel de celui-ci avec Clonostachys compactiuscula ATCC 38009, ou ses mutants, ou une hydrolase dérivée de celui-ci, et récupération du produit.

2. Procédé selon la revendication 1, dans lequel le sel est le sel d'ammonium.

3. Procédé selon la revendication 1, dans lequel l'hydrolase est sous forme purifiée et immobilisée sur une colonne, et le composé de formule 1 est mis en contact avec elle par passage à travers ladite colonne.

4. Forme pratiquement pure de l'enzyme hydrolase produite par Clonostachys compactiuscula ATCC 38009, et ses mutants, qui est apte à mettre en oeuvre un procédé selon la revendication 1.

5. Procédé selon la revendication 1, comprenant en outre une lactonisation de l'acide triol de formule 2 pour procurer un lactone diol de formule structurale 3:

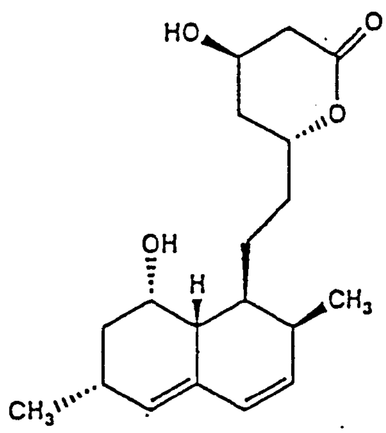

3 lactone diol